# EUROPEAN PATENT APPLICATION

(11) **EP 4 088 698 A1**
(43) Date of publication of application: **16.11.2022**
(21) Application number: 21767757.4
(22) Date of filing: 09.03.2021
(51) Int. Cl.: A61F 13/15, A61F 13/511, A61F 13/512, A61F 13/537, A61F 13/539

(54) **DISPOSABLE ABSORBENT ARTICLE**

(30) Priority: 13.03.2020 JP 2020044429
(71) Applicant: Daio Paper Corporation, Ehime 799-0492 (JP)
(72) Inventor: TAKAGI, Yurika, Shikokuchuo-shi, Ehime 799-0431 (JP)
(74) Representative: Williams Powell
(86) International application number: PCT/JP2021/009193
(87) International publication number: WO 2021/182445

(57) **Abstract**

To provide a disposable absorbent article in which a top sheet and an intermediate sheet on a back surface side are bonded to each other without hindering visibility of openings in the top sheet.

An absorbent article includes: a top sheet constituting a usage-side surface; a liquid impervious sheet disposed on a back surface side; and an absorbent element interposed between the top sheet and the liquid impervious sheet. The top sheet is a perforated nonwoven fabric in which a large number of openings 14 penetrating the nonwoven fabric from a front surface to a back surface are formed at intervals in a front-back direction and a width direction of a product. A liquid pervious intermediate sheet is bonded to a back surface side of the top sheet at a large number of bonding points 15 by thermal bonding or ultrasonic bonding. The openings 14 each have an area of 1 to 30 mm². The bonding points 15 each have an area of 0.12 to 20 mm², the area being smaller than the area of the opening 14.

## Description

### Technical Field

The present invention relates to a disposable absorbent article such as a disposable diaper.

### Background Art

A disposable absorbent article, particularly a disposable diaper often causes roughness of the skin of a wearer, particularly, rash disadvantageously.

Examples of a cause for this disadvantage include friction with the skin at the time of wearing the diaper, and stimulation received by the skin from a body fluid and excreta (urine and loose stool) due to wearing the diaper for a long time.

In particular, the effect of stimulation due to contact of the skin with loose stool for a long time is large. In order to suppress this, the diaper needs to quickly absorb loose stool into an absorber. If the diaper can quickly absorb loose stool into the absorber, it is useful not only in reducing stimulation received by the skin but also in preventing leakage from a leg portion or a dorsal portion.

A first cause for hindering absorption of loose stool through a top sheet is that when a loose stool component passes through the top sheet, a loose stool component that cannot pass through the top sheet remains on a surface portion of fibers constituting the top sheet, and the top sheet is clogged. A second cause for hindering absorption of loose stool through the top sheet is that a defecation speed exceeds an absorption speed of the diaper, and loose stool cannot be absorbed and remains on the top sheet.

Therefore, it is very important for the diaper to quickly absorb loose stool into an absorber.

Patent Literature 1 discloses that a skin care agent, particularly a skin care agent containing a diamide derivative is disposed in a top sheet between so-called gather cuffs on both sides of a diaper in a width direction.

In particular, in a diaper worn by an infant, the skin of the infant is sensitive, and so-called diaper rash easily occurs.

### Citation List

### Patent Literature

Patent Literature 1: JP 2018-102836 A

### Summary of Invention

### Technical Problem

However, there is a limit to suppressing diaper rash only by disposing the skin care agent. When a loose stool component does not quickly pass through the top sheet, it is difficult to suppress diaper rash.

On the other hand, it is known to form openings in the top sheet in order to enhance liquid perviousness or liquid perviousness of a loose stool component.

However, when an attempt is made to bond the perforated top sheet having openings formed therein to an intermediate sheet on a back surface side, for example, a so-called second sheet using, for example, a hot melt adhesive, there is a concern that the hot melt adhesive may ooze out to a top sheet surface side through the opening and affect the skin.

Therefore, a main object of the present invention is to provide a disposable absorbent article in which a top sheet and an intermediate sheet on a back surface side are bonded to each other without hindering visibility of openings in the top sheet.

### Solution to Problem

A representative aspect of the present invention solving the above problem is as follows.

### <Representative aspect>

A disposable absorbent article including: a top sheet constituting a usage-side surface; a liquid impervious sheet disposed on a back surface side; and an absorbent element interposed between the top sheet and the liquid impervious sheet, wherein
the top sheet is a perforated nonwoven fabric in which a large number of openings penetrating the nonwoven fabric from a front surface to a back surface are formed at intervals in a front-back direction and a width direction of a product,
a liquid pervious intermediate sheet is disposed on a back surface side of the top sheet,
the top sheet and the intermediate sheet are bonded to each other at a large number of bonding points by thermal bonding or ultrasonic bonding,
the openings each have an area of 1 to 30 mm²,
the bonding points each have an area of 0.12 to 20 mm², the area being smaller than the area of the opening,
a separation distance between the openings in a width direction is different from a separation distance between the bonding points in the width direction by 0.2 mm or more, and
a separation distance between the openings in a front-back direction is different from a separation distance between the bonding points in the front-back direction by 0.2 mm or more.

### Advantageous Effects of Invention

The present invention brings about an advantage that the top sheet and the intermediate sheet on the back surface side are bonded to each other while an influence on the skin is prevented.

### Brief Description of Drawings

Fig. 1 is a plan view illustrating an inner surface of a tape-type disposable diaper in an unfolded state.
Fig. 2 is a plan view illustrating an outer surface of the tape-type disposable diaper in an unfolded state.
Fig. 3 is a cross-sectional view taken along line 3-3 in Fig. 1.
Fig. 4 is a cross-sectional view taken along line 4-4 in Fig. 1.
Fig. 5(a) is a cross-sectional view taken along line 5a-5a in Fig. 1, Fig. 5(b) is a cross-sectional view taken along line 5b-5b in Fig. 1, and Fig. 5(c) is a cross-sectional view taken along line 5c-5c in Fig. 1.
Fig. 6 is a plan view of an arrangement example of openings.
Fig. 7 is a plan view of another arrangement example of the openings.
Fig. 8 illustrates an embodiment indicating a relationship between openings and bonding points, in which Fig. 8(a) is a plan view illustrating a positional relationship between the openings and the bonding points, and Fig. 8(b) is a plan view illustrating individual positions of the openings and the bonding points.
Fig. 9 is a plan view of another arrangement example of the bonding points.
Fig. 10 illustrates a reference example indicating a relationship between openings and bonding points, in which Fig. 10(a) is a plan view illustrating a positional relationship between the openings and the bonding points, and Fig. 10(b) is a plan view illustrating individual positions of the openings and the bonding points.
Fig. 11 is a plan view of an application example of a moisturizing agent.
Fig. 12 is a schematic explanatory view of a transition form of the moisturizing agent.

### Description of Embodiments

Hereinafter, an embodiment of the present invention will be described in detail with reference to the attached drawings. A dotted pattern portion in the cross-sectional views illustrates an adhesive as a bonding means for bonding constituent members located on a front surface side and a back surface side, and is formed by applying a hot melt adhesive by solid application, bead application, curtain application, summit application, spiral application, pattern coating (transfer of a hot melt adhesive by a letterpress method), or the like. Alternatively, a fixing portion of an elastically stretchable member is formed, instead of this or in addition to this, by application of an elastically stretchable member to an outer peripheral surface by a comb gun, SureWrap application, or the like. Examples of the hot melt adhesive include an EVA-based agent, a pressure sensitive adhesive rubber-based agent (elastomer-based agent), a polyolefin-based agent, and a polyester/polyamide-based agent, and these can be used without particular limitation. As a bonding means for bonding constituent members to each other, a means by material welding such as heat sealing or ultrasonic sealing can also be used.

### <Example of tape-type disposable diaper>

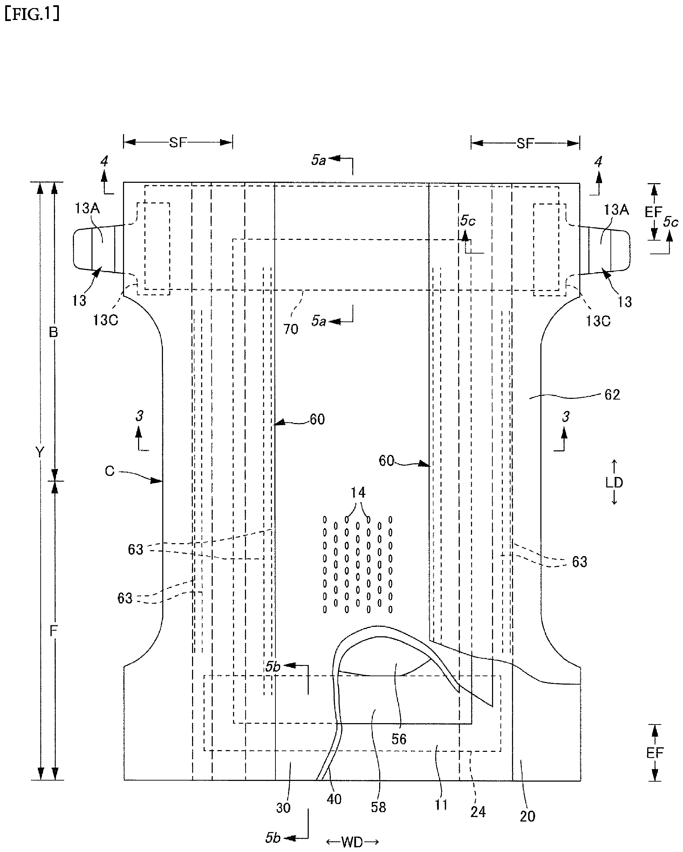

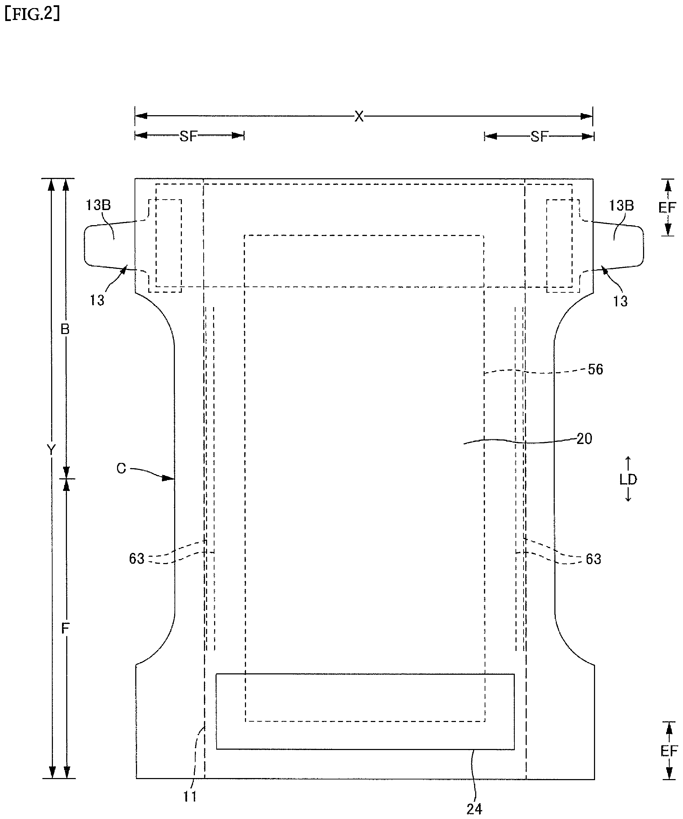

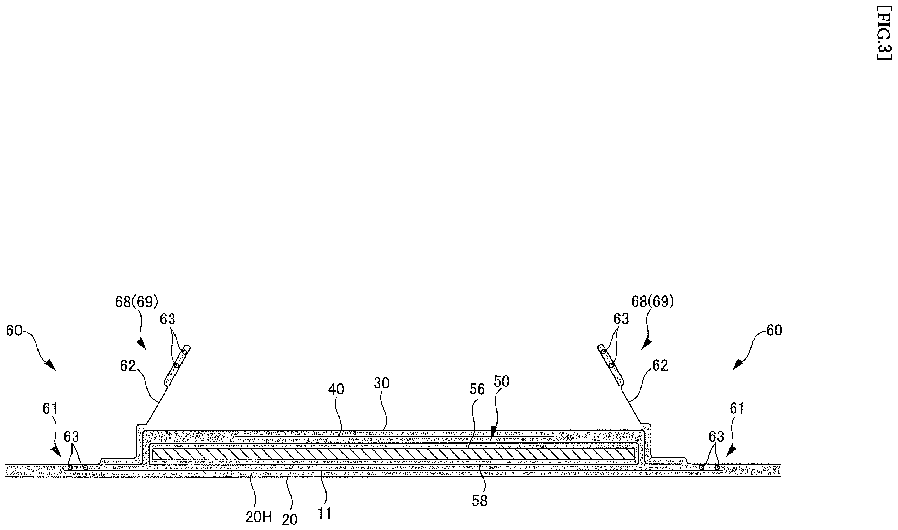

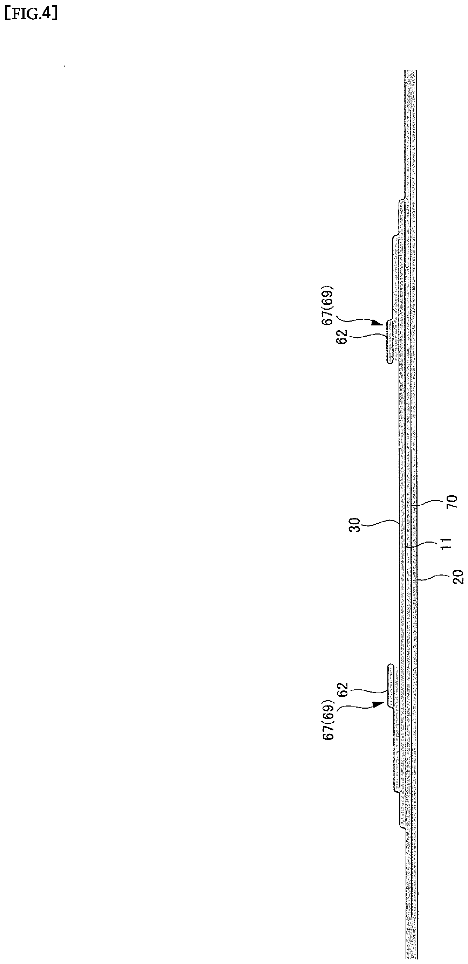

Figs. 1 to 5 illustrate an example of a tape-type disposable diaper as the disposable absorbent article of the present invention, in which a reference character X indicates the maximum width of the diaper excluding a fastening tape, and a reference character Y indicates the maximum length of the diaper. This tape-type disposable diaper includes an absorber 56 extending from a ventral side to a dorsal side, a liquid pervious top sheet 30 covering a front surface side of the absorber 56, a liquid impervious sheet 11 covering a back surface side of the absorber 56, a ventral side end flap portion EF and a dorsal side end flap portion EF that are portions extending to a front side and a back side of the absorber 56, respectively, and not including the absorber 56, and a pair of side flap portions SF extending laterally beyond side edges of the absorber 56. A constricted portion along a periphery of the leg is formed in the middle of the side flap portion SF in the front-back direction, and a fastening tape 13 is disposed on the dorsal side of the constricted portion.

The back surface of the liquid impervious sheet 11 is covered with a cover nonwoven fabric 20. The cover nonwoven fabric 20 extends to a peripheral edge of the diaper, and the liquid impervious sheet 11 extends to front-back edges of the diaper in the front-back direction and extends to a portion between a side edge of the absorber 56 and a side edge of the cover nonwoven fabric 20 in the width direction. However, the cover nonwoven fabric 20 may be disposed in only a part in the front-back direction, in only a part in the width direction, or in a part in the front-back direction and a part in the width direction as necessary. For example, when a part of the liquid impervious sheet 11 is covered with another material such as a gather nonwoven fabric, the cover nonwoven fabric 20 does not have to be disposed in the part.

The top sheet 30 and the liquid impervious sheet 11 are rectangular in the illustrated example, and each have a size slightly larger than the absorbent element 50 in each of the front-back direction and the width direction. A peripheral edge portion projecting from a side edge of the absorbent element 50 in the top sheet 30 is bonded to a peripheral edge portion projecting from a side edge of the absorbent element 50 in the liquid impervious sheet 11 with a hot melt adhesive or the like.

The absorbent element 50 includes the absorber 56 and a wrapping sheet 58 wrapping the absorber, and can be interposed between the top sheet 30 and the liquid impervious sheet 11. An intermediate sheet 40 can be disposed between the top sheet 30 and the absorbent element 50.

The intermediate sheet 40 in the illustrated form is disposed at the center so as to be shorter than the width of the absorbent element 50, but may be disposed over the maximum width. The front-back direction length of the intermediate sheet 40 may be the same as the maximum length of the diaper, may be the same as the length of the absorbent element 50, or may be within a short length range centered on a liquid receiving region. Furthermore, a discoloring indicator that comes into contact with a liquid of excreta can also be disposed.

Side gathers 60 are disposed on both sides of a surface of the tape-type disposable diaper in the width direction. Each of the side gathers 60 includes a first portion 61 (planar gather portion) disposed in each side flap portion SF and a second portion 69 (three-dimensional gather portion) protruding upward from each side portion of the top sheet 30.

More specifically, a belt-shaped gather nonwoven fabric 62 having a length equal to the maximum length Y of the diaper extends from the first portion 61 to the second portion 69. In the first portion 61, the gather nonwoven fabric 62 is bonded to the cover nonwoven fabric 20 with a hot melt adhesive or the like. Between these nonwoven fabrics, one or more elastically stretchable gather members 63 in the front-back direction LD are fixed in a stretched state at intervals in the width direction WD, and the first portion 61 contracts in the front-back direction LD by a contraction force thereof to form a planar gather in contact with a periphery of the leg.

In addition, the gather nonwoven fabric 62 has an extending portion extending from the first portion 61 as a root portion toward the central side in the width direction WD, and at least this extending portion is folded back at a tip to have a two-layer structure. Both end portions of the extending portion in the front-back direction LD are fallen portions 67 fixed to the top sheet 30, and an intermediate portion in the front-back direction LD located therebetween is a non-fixed free portion 68. One or more elastically stretchable gather members 63 in the front-back direction LD are fixed to the free portion 68 in a stretched state at intervals in the width direction WD, and the free portion 68 of the second portion 69 contracts in the front-back direction LD by a contraction force thereof to form a three-dimensional gather in contact with a periphery of the leg.

The fastening tape 13 in the illustrated embodiment includes: a tape attachment portion 13C fixed to a side portion of the diaper; a sheet base material forming a tape main unit portion 13B protruding from the tape attachment portion 13C; and a ventral side engagement portion 13A disposed in a width direction intermediate portion of the tape main unit portion 13B in the sheet base material, and a tip side portion of the engagement portion 13A is a tab part. The tape attachment portion 13C of the fastening tape 13 is sandwiched between the gather nonwoven fabric 62 forming an inner layer and the cover nonwoven fabric 20 forming an outer layer in the side flap portion, and is bonded to these nonwoven fabrics 62 and 20 with a hot melt adhesive. The engagement portion 13A is bonded to an inner surface of the tape main unit portion 13B with an adhesive.

As the engagement portion 13A, a hook member (male member) of a mechanical fastener (hook and loop fastener) is suitable. The hook member has a large number of engaging projections on an external surface side thereof. Examples of the shapes of the engaging projections include (A) tick shape, (B) J shape, (C) mushroom shape, (D) T shape, and (E) double J shape (a shape in which the J-shaped ones are connected to each other back to back), but any one of these shapes may be used. Of course, a pressure sensitive adhesive material layer can be disposed as an engagement portion of the fastening tape 13.

As the sheet base material forming a portion from the tape attachment portion 13C to the tape main unit portion 13B, a nonwoven fabric, a plastic film, a polylaminated nonwoven fabric, paper, or a composite material thereof can be used.

At the time of wearing the diaper, in a state where the side flap portion SF on the dorsal side is superimposed on an outer side of the side flap portion SF on the ventral side, the fastening tape 13 is engaged at an appropriate position on an outer surface of the ventral side portion F. The position and the size of the engaging location of the fastening tape 13 can be arbitrarily determined.

A target sheet 24 having a target for facilitating engagement is preferably disposed at the engaging location of the fastening tape 13 in the ventral side portion F. When the engagement portion 13A is a hook member, as the target sheet 24, a film type sheet having a film layer and an engagement layer disposed on the entire outer surface of the film layer and detachably engaged with the hook of the engagement portion 13A can be suitably used. As the engagement layer in this case, in addition to a form in which a net-like body knitted with a yarn and having a loop is attached on the film layer, a form in which a nonwoven fabric layer of a thermoplastic resin is attached on a film layer by intermittent ultrasonic sealing and fibers of the nonwoven fabric form a loop is known, but either of these forms can be suitably used. In addition, a film-less type target tape obtained by embossing a nonwoven fabric of a thermoplastic resin and having no film layer can also be used. In these target tapes, the hook of the fastening tape 13 is entangled with or caught in the loop, whereby the fastening tape 13 is connected.

When the engagement portion 13A is a pressure sensitive adhesive material layer, a product obtained by performing a peeling process on a surface of a sheet base material, the surface being formed of a smooth plastic film with high pressure sensitive adhesiveness, can be used.

In addition, when the engaging location of the fastening tape 13 in the ventral side portion F is formed of a nonwoven fabric, for example, when the cover nonwoven fabric 20 in the illustrated embodiment is formed of a nonwoven fabric and the engagement portion 13A of the fastening tape 13 is formed of a hook member, the target sheet 24 can be omitted, and the hook member can be entangled with the nonwoven fabric of the cover nonwoven fabric 20 to be engaged. In this case, the target sheet 24 may be disposed between the cover nonwoven fabric 20 and the liquid impervious sheet 11.

The end flap portion EF is a portion that extends to each of the front side and the back side of the absorbent element 50 and does not have the absorber 56. The front extending portion is the ventral end flap portion EF, and the back extending portion is the dorsal end flap portion EF.

The length of the dorsal side end flap portion EF in the front-back direction is preferably the same as or shorter than the length of an attachment portion of the fastening tape 13 in the front-back direction for the above-described reason. In addition, when the diaper dorsal end portion and the absorbent element 50 are too close to each other, a gap is likely to be generated between the diaper dorsal end portion and the body surface due to the thickness and elasticity of the absorbent element 50. Therefore, the length is preferably 10 mm or more.

The length of each of the ventral side end flap portion EF and the dorsal side end flap portion EF in the front-back direction is preferably about 5 to 20% of the length Y of the entire diaper in the front-back direction. In an infant diaper, it is appropriate to set the length of each of the ventral side end flap portion EF and the dorsal side end flap portion EF in the front-back direction to 10 to 60 mm, particularly 20 to 50 mm.

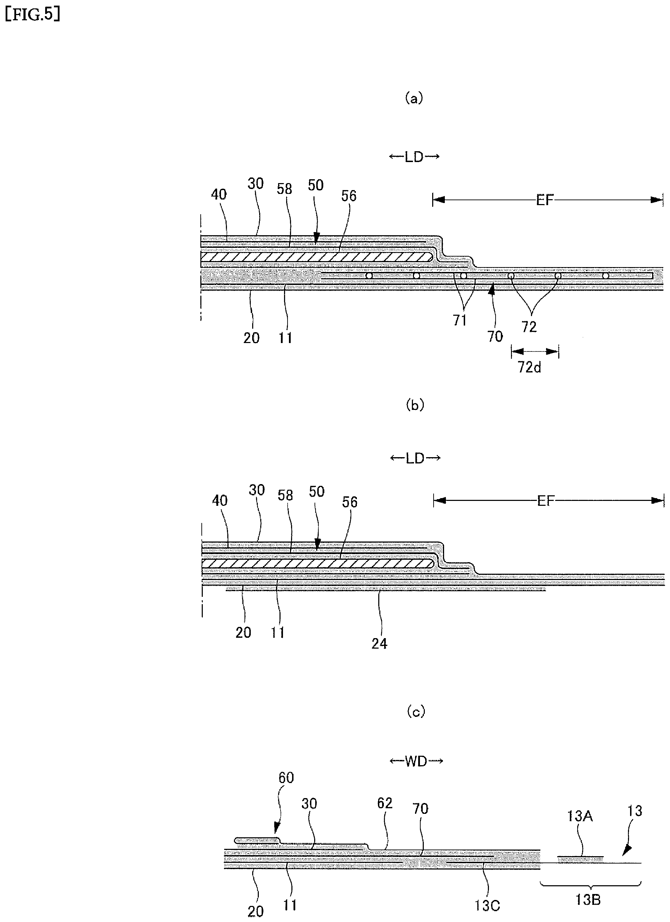

In order to improve fitting on the dorsal side of the diaper, specifically, as in the form illustrated in Fig. 5, an elastically stretchable member that elastically stretches and contracts in the width direction, particularly, a belt-shaped dorsal side stretchable sheet 70 is preferably disposed between both the fastening tapes 13. Each end portion of the dorsal side stretchable sheet 70 preferably extends to a site where the dorsal side stretchable sheet 70 overlaps the attachment portion of both the fastening tapes 13, but may be separated from the attachment portion of both the fastening tapes 13 on the central side in the width direction. The size of the dorsal side stretchable sheet 70 in the front-back direction is preferably within a range of about plus or minus 20% with respect to the size of the attachment portion of the fastening tape 13 in the front-back direction. In addition, when the dorsal side stretchable sheet 70 is disposed so as to overlap a boundary line between the dorsal side end flap portion EF and the absorbent element 50 as illustrated in the drawing, the dorsal side end portion of the absorbent element 50 is firmly pressed against the body, which is preferable.

As the dorsal side stretchable sheet 70, a sheetshaped elastic member such as a rubber sheet may be used, but a nonwoven fabric or paper is preferably used from a viewpoint of air permeability. In this case, a sheetshaped elastic member having air permeability, such as a stretchable nonwoven fabric, can be used. However, as illustrated in Fig. 5(a), a member obtained by sticking two sheet base materials 71 formed of a nonwoven fabric or the like to each other with an adhesive such as a hot melt adhesive and fixing an elastically stretchable member 72 in a perforated sheet shape, a net shape, an elongated shape (a thread shape, a string shape, or the like), or the like between the sheet base materials 71 in a stretched state in the width direction is suitably used. As the sheet base material 71 in this case, a similar material to that of the cover nonwoven fabric 20 can be used. The elastically stretchable member 72 preferably has a stretch rate of about 150 to 250%. In addition, when an elongated (thread-shaped, string-shaped, or the like) member is used as the elastically stretchable member 72, it is preferable to dispose about 5 to 15 members each having a fineness of 420 to 1120 dtex at an interval 72d of 3 to 10 mm.

As illustrated in Fig. 5(a), it is preferable to dispose a part of the elastically stretchable member 72 so as to cross the absorbent element 50 because fitting of the absorbent element 50 is improved. However, in this case, when a contraction force is prevented from acting on a part or the whole of a portion where the elastically stretchable member 72 overlaps the absorbent element 50 by means of cutting or the like, the dorsal side end portion of the absorbent element 50 does not contract in the width direction, and therefore fitting is further improved.

Note that the elastically stretchable member 72 may be fixed over the maximum length of the sheet base material 71 in the width direction of the diaper, but in order to prevent shrinkage or turn-up at the time of attachment to the diaper main body, it is preferable to prevent a contraction force from acting or not to dispose the elastically stretchable member 72 in a range of about 5 to 20 mm of an end portion of the diaper in the width direction. When the elastically stretchable member 72 is not disposed, a frill that does not come into contact with the skin can be formed, and air permeability is improved.

In the illustrated embodiment, the dorsal side stretchable sheet 70 is sandwiched between the gather nonwoven fabric 62 and the cover nonwoven fabric 20 on both sides of the liquid impervious sheet 11 in the width direction, and sandwiched between the liquid impervious sheet 11 and the absorbent element 50 at a site where the dorsal side stretchable sheet 70 overlaps the liquid impervious sheet 11. However, the dorsal side stretchable sheet 70 may be disposed between the liquid impervious sheet 11 and the cover nonwoven fabric 20, may be disposed on an outer surface of the cover nonwoven fabric 20, or may be disposed between the top sheet 30 and the absorbent element 50.

Furthermore, the dorsal side stretchable sheet 70 may be disposed on the top sheet 30, and in this case, may be disposed on the gather nonwoven fabric 62 on both sides of the liquid impervious sheet 11 in the width direction. When the cover nonwoven fabric 20 is formed by stacking a plurality of sheet base materials, the entire dorsal side stretchable sheet 70 may be disposed between the sheet base materials of the cover nonwoven fabric 20.

### <Basic configuration of the present invention>

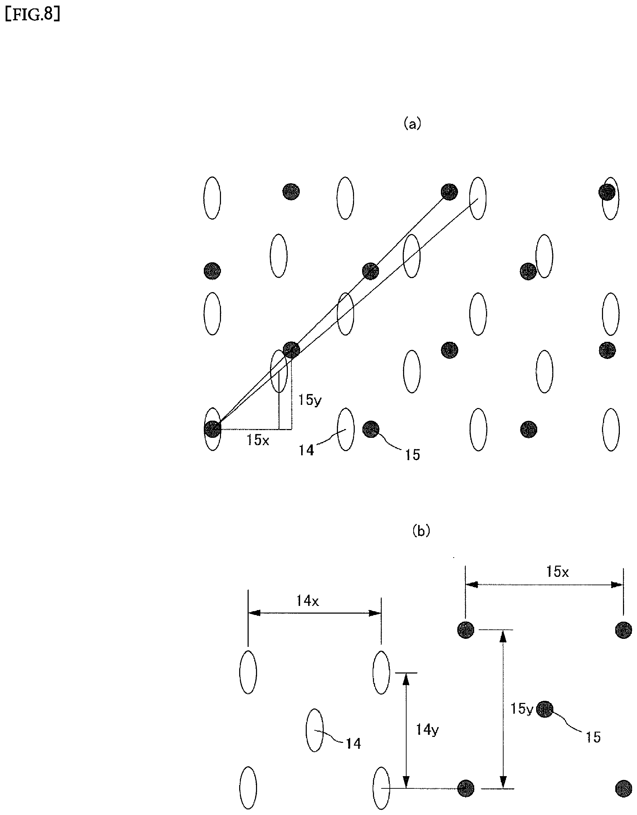

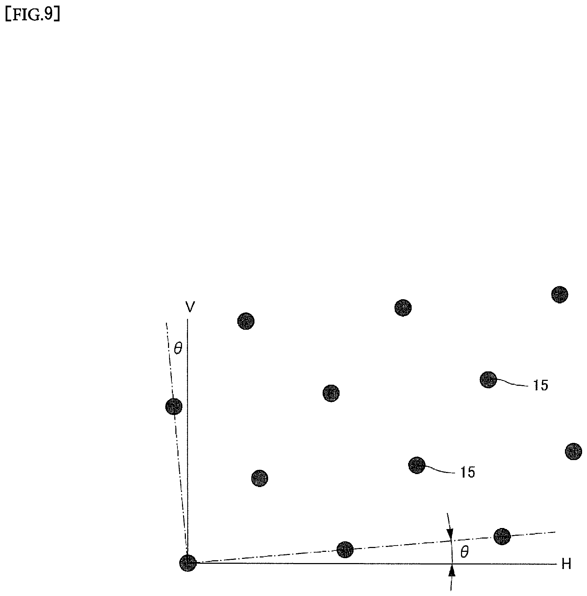

If explanatory reference characters in the above embodiment are referred to, the disposable diaper exemplified as the disposable absorbent article of the present invention is a disposable diaper including: the top sheet 30 constituting a usage-side surface; the liquid impervious sheet 11 disposed on a back surface side; and the absorbent element 50 interposed between the top sheet 30 and the liquid impervious sheet 11, in which
the top sheet 30 is a perforated nonwoven fabric in which a large number of openings 14 penetrating the nonwoven fabric from a front surface to a back surface are formed at intervals,
the liquid pervious intermediate sheet 40 is disposed on a back surface side of the top sheet 30, and
the top sheet 30 and the intermediate sheet 40 are bonded to each other at a large number of bonding points 15 (see Figs. 8 and 9) by thermal bonding or ultrasonic bonding.

In the basic configuration of the present invention, when the top sheet 30 is a perforated nonwoven fabric in which the large number of openings 14 penetrating the nonwoven fabric from a front surface to a back surface are formed at intervals, a loose stool component can be quickly absorbed into the absorbent element 50 having the absorber 56 through the openings 14, which is useful not only for reducing stimulation received by the skin due to the loose stool component remaining on a surface of the top sheet 30 to suppress diaper rash but also for preventing leakage from a leg portion or a dorsal portion.

### (Opening)

The top sheet 30 is a perforated nonwoven fabric having a large number of openings 14. Although an exact reason is not clear, it is considered that a function of reducing a frictional force with the skin of a wearer is also exhibited because a contact area with the skin of the wearer is reduced by the large number of openings 14 as compared with a nonwoven fabric having no openings. As a result, it is considered that when the disposable diaper is worn, the top sheet slides with respect to the skin according to a change in the posture of a wearer, contact with the skin of the wearer is secured, and a leakage preventing effect is enhanced.

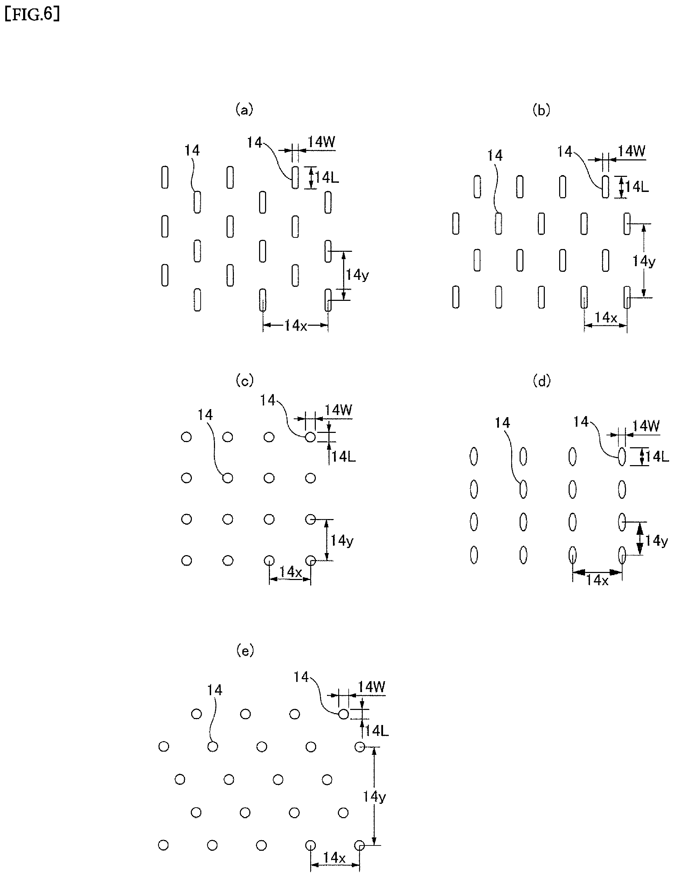

In addition to the long hole shape as illustrated in Figs. 6(a) and 6(b), the shape of each of the openings 14 can be an arbitrary shape such as a perfect circle as illustrated in Figs. 6(c) and 6(e), an ellipse as illustrated in Fig. 6(d), a polygon such as a triangle, a rectangle, or a rhombus, a star shape, a cloud shape, or the like.

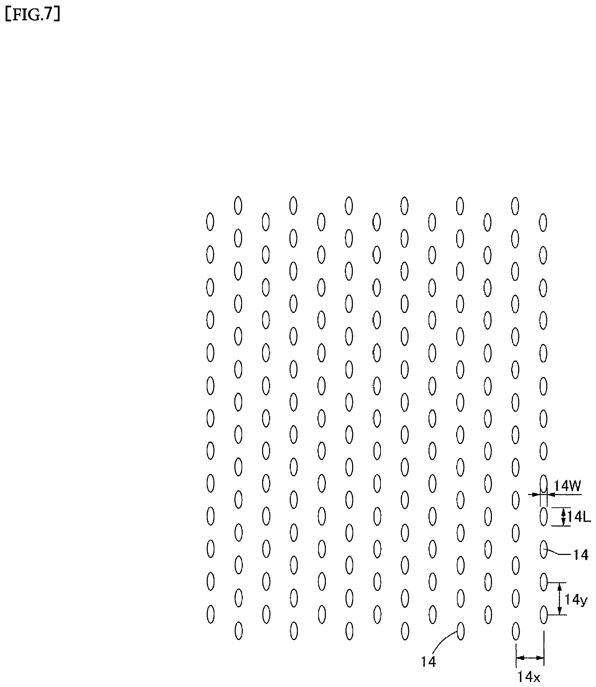

In particular, among the shapes illustrated in Figs. 1, 6(a) to 6(e), and 7, a perfect circle, an ellipse, a quadrilateral (including a square and a rectangle, and having corners cut off by an arc or the like (rounded corners)), and an intermediate form between a rectangle and an ellipse are desirable.

The size of each of the openings 14 according to the present invention is not particularly limited. However, when the size is exemplified with reference to Fig. 6, a maximum size 14L in the front-back direction LD is preferably 0.3 to 6.0 mm, particularly preferably 1.2 preferably 4.0 mm, and a maximum size 14W in the width direction WD is preferably 0.3 to 6.0 mm, particularly preferably 0.5 to 1.5 mm. When the shape of the opening 14 is a long shape in one direction (a shape in which a maximum length in one direction is longer than a maximum length in a direction orthogonal thereto) such as a long hole shape, an elliptical shape, or a rectangular shape, a maximum size in the longitudinal direction is preferably 1.2 to 2.5 times a maximum size in a direction orthogonal thereto. In addition, when the shape of the opening 14 is long in one direction, it is desirable that the longitudinal direction of the opening 14 is the front-back direction LD, but may be the width direction WD or an oblique direction.

The area and area ratio of each of the openings 14 can be set mainly in consideration of, for example, perviousness of a loose stool component, and the area is set to 1 to 30 mm². In particular, the area is preferably 2.5 to 10.0 mm², and the area ratio is preferably 1.0 to 15.0% (particularly 5.0 to 10.0%).

The area of an ellipse can be obtained by πab when the length of the major axis is represented by a and the length of the minor axis is represented by b. The area of a rounded rectangle (rectangle with rounded corner) can be obtained by ab + (π - 4) r² when the length is represented by a, the width is represented by b, and the radius of the rounded corner is represented by r.

### (Intermediate sheet: second sheet)

In order to quickly transfer liquid that has passed through the top sheet 30 to the absorber, an intermediate sheet (also referred to as "second sheet") 40 having a higher liquid permeation speed than the top sheet 30 is disposed. This intermediate sheet 40 not only quickly transfers liquid to the absorber to enhance absorption performance by the absorber, but also prevents a "returning" phenomenon of the absorbed liquid from the absorber, and can make a top surface of the top sheet 30 dry all the time.

The intermediate sheet 40 is bonded to the top sheet 30 at a large number of bonding points by thermal bonding or ultrasonic bonding in order to prevent deviation thereof in the sheet extending direction and separation from the top sheet 30, to receive liquid that has passed through the top sheet 30, and to quickly transfer the liquid to the absorber.

When the intermediate sheet 40 and the top sheet 30 are bonded to each other with a hot melt adhesive, the hot melt adhesive may ooze out to a surface side of the top sheet 30 through the openings 14. As a result, not only the skin may be affected, but also the oozed hot melt adhesive may contaminate manufacturing apparatuses in a product manufacturing process, leading to an operational trouble.

On the other hand, according to the bonding form by thermal bonding or ultrasonic bonding, it is possible to prevent occurrence of the above-described problem.

On the other hand, it has become clear that it is necessary to consider a positional relationship between the bonding points 15 (see Figs. 8 and 9) and the openings 14 by thermal bonding or ultrasonic bonding.

That is, formation of the openings 14 has, in addition to retention of the liquid pervious function, a function of appealing to a consumer to visually confirm presence of the openings 14 when the product is unfolded, thereby increasing a desire of purchase.

However, if the position of the opening 14 and the position of the bonding point 15 coincide with each other or the bonding point 15 is close to the position of the opening 14, the opening 14 portion is integrated with the intermediate sheet 40. As a result, liquid permeation from the opening 14 is hindered, and a periphery of the opening 14 of the top sheet 30 is flattened by the integration, which makes it difficult to visually recognize the opening 14 (visibility of a shade provided by a concave around the opening 14 is impaired).

Note that in general, the opening 14 is formed by puncturing one surface of the sheet with a sharp pin or the like. As a result, a three-dimensional concave is formed in a peripheral edge portion of the opening 14, and a shade of the concave is visually recognized by a consumer. Presence of the opening 14 is thereby confirmed.

Therefore, it is desirable that the bonding point 15 between the top sheet 30 and the intermediate sheet 40 by thermal bonding or ultrasonic bonding does not coincide with the position of the opening 14 if possible.

Specifically, a separation distance 14x between the openings 14 in the width direction is different from a separation distance 15x between the bonding points 15 in the width direction illustrated in Figs. 8 and 9 by 0.2 mm or more, and a separation distance 14y between the openings 14 in the front-back direction is different from a separation distance 15y between the bonding points 15 in the front-back direction by 0.2 mm or more.

That is, this means that a "difference in the separation distance" in the width direction WD between the opening 14 and the bonding point 15 is 0.2 mm or more, and a "difference in the separation distance" in the front-back direction LD between the opening 14 and the bonding point 15 is 0.2 mm or more.

The "separation distance" between the openings 14 in the width direction refers to a pitch between center points of adjacent openings 14 in the width direction in the opening 14 row in the width direction. It is desirable that the "separation distance" in the width direction is 3.0 to 9.0 mm, particularly 3.5 to 6.5 mm.

The "separation distance" in the front-back direction refers to a pitch between center points of adjacent openings 14 in the front-back direction in the opening 14 row in the front-back direction. It is desirable that the "separation distance" in the front-back direction is 2.5 to 9.0 mm, particularly 3.5 to 6.5 mm.

The "separation distance" between the bonding points 15 in the width direction refers to a pitch between center points of adjacent bonding points 15 in the width direction in the bonding point 15 row in the width direction. It is desirable that the "separation distance" in the width direction is 2.0 to 7.0 mm, particularly 3.5 to 6.0 mm.

The "separation distance" in the front-back direction refers to a pitch between center points of adjacent bonding point 15 rows in the front-back direction in the bonding point 15 row in the front-back direction. It is desirable that the "separation distance" in the front-back direction is 2.5 to 8.0 mm, particularly 3.2 to 5.0 mm.

The bonding point 15 has an area of 0.12 to 20 mm², the area being smaller than the area of the opening 14.

The area of the bonding point 15 defines a bonding strength between the top sheet 30 and the intermediate sheet 40 in relation to the above-described separation distance between the bonding points 15 in each of the width direction and the front-back direction.

The area of the bonding point 15 is 0.12 to 20 mm², and particularly desirably 0.25 to 5 mm² from this viewpoint.

When the area of the bonding point 15 is smaller than the area of the opening 14, even if the bonding point 15 coincides with the position of the opening 14, a periphery of the opening 14 is flattened, and an influence of difficulty in visually recognizing the opening 14 (visibility of a shade provided by a concave around the opening 14 is impaired) is small.

When the area of the bonding point 15 is excessively small, it is difficult to obtain a required bonding strength.

It is desirable that the separation distance between the openings 14 in the opening 14 row and the separation distance between the bonding points 15 in the bonding point 15 row are desirably constant. If the separation distances are not constant, when the top sheet 30 is visually recognized in a wide range, the density of coincidence points where the openings 14 and the bonding points 15 coincide with each other varies, which may impart an uncomfortable feeling in visual recognition to a consumer.

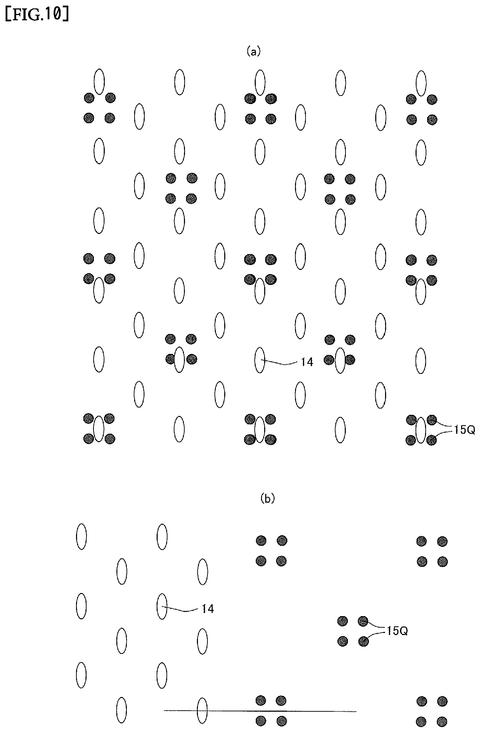

On the other hand, for example, as illustrated in Fig. 10(b), when four bonding points 15Q are formed at a density concentrated around an intersection of an oblique lattice with respect to the opening 14 group having a regular arrangement (therefore, a separation distance between adjacent bonding points is not regular and not constant), there is an advantage that a bonding strength in the vicinity of the four bonding points 15Q is increased by an interaction of the four bonding points 15Q, and a separation distance between the groups each including the four bonding points 15Q can be increased. However, as illustrated in Fig. 10(a), when the group including the four bonding points 15Q is located at the same position as or in the vicinity of the opening 14, the group of the bonding points 15Q surrounds the opening 14, a periphery of the opening 14 is flattened, and it is difficult to visually recognize the opening 14 (visibility of a shade provided by a concave around the opening 14 is impaired).

As illustrated in Fig. 8, repetitive rows of the bonding points 15 can be arranged along the width direction WD and the front-back direction LD in the width direction and the front-back direction, respectively.

Alternatively, as illustrated in Fig. 9, the repetitive rows of the bonding points 15 can be inclined with respect to a width direction line H and a front-back direction line V at an angle θ. By disposing the repetitive rows of the bonding points 15 at an angle θ (3 to 6 degrees is desirable), a coincidence probability with respect to the opening 14 row in each of the width direction and the front-back direction can be reduced, and as a result, hindrance of visibility of the opening 14 group can be prevented.

The embodiment of Fig. 8 is an example in which the "the separation distance" in the width direction WD for the bonding point 15 is longer by 0.2 mm or more than that for the opening 14, and the "the separation distance" in the front-back direction LD for the bonding point 15 is longer by 0.2 mm or more than that for the opening 14.

Also in this embodiment, a coincidence probability with respect to the opening 14 row in each of the width direction and the front-back direction can be reduced, and as a result, hindrance of visibility of the opening 14 group can be prevented.

When the difference in the separation distance is adopted, the length may be "decreased" instead of "increased".

It is desirable that the bonding strength between the top sheet 30 and the intermediate sheet is 30 to 100 gf. When the bonding strength is small, peeling is likely to occur, and an excessive bonding strength leads to deterioration in texture.

The bonding strength can be measured using a tensile testing machine (AUTOGRAPH AG-Xplus manufactured by SHIMADZU CORPORATION). As a measurement method, a sample cut into 40 mm × 300 mm is prepared, 100 mm of the sample in a longitudinal direction is peeled off in advance to form a peeling start part, the test piece is set such that the test piece does not sag at 23°C, a length of 150 mm from the peeling start part is peeled off with the tensile testing machine at a tensile speed of 300 mm/min, and measurement is performed to confirm the bonding strength.

### (Moisturizing agent)

Suitably, when a moisturizing agent M mainly containing glycerin is applied to an outer surface of the top sheet 30 (at least the outer surface portion of the top sheet 30 contains the moisturizing agent M), the moisturizing agent M not only protects the skin of a wearer but also exhibits a function of reducing a frictional force with the skin of the wearer.

As a result, when the disposable diaper is worn, the top sheet slides with respect to the skin according to a change in the posture of a wearer, contact with the skin of the wearer is secured, and a leakage preventing effect is enhanced.

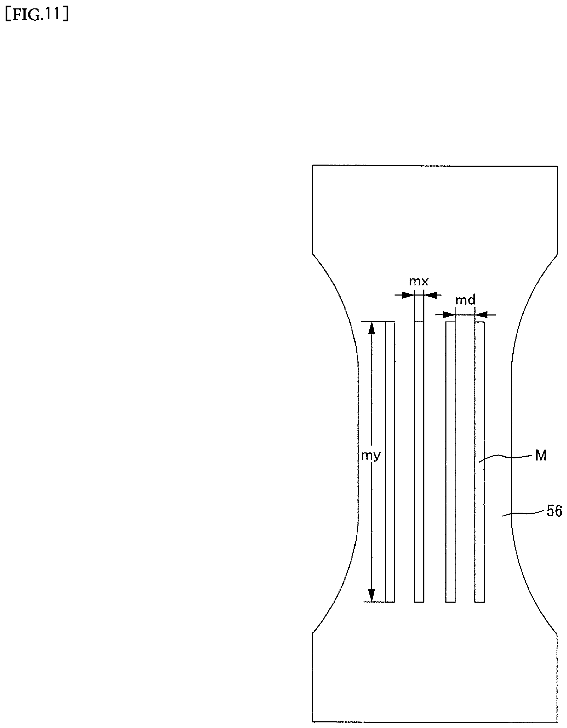

For example, as illustrated in Fig. 11, the moisturizing agent M can be applied in the front-back direction at intervals in the width direction. As this application, for example, the moisturizing agent may be discharged from application nozzles disposed at intervals in the width direction in a manufacturing process, or the moisturizing agent may be applied by a transfer method.

For example, an application width mx of the moisturizing agent M illustrated in Fig. 11 can be 5 to 30 mm, an interval md can be 5 to 30 mm, and an application length my in the front-back direction can be 50 mm to the front-back length of a product.

The moisturizing agent M may be applied to the front-back length of a product in the front-back direction. In addition, it is desirable that the moisturizing agent M is applied mainly to a crotch portion where the effect according to the present invention is remarkably exhibited and is not applied to a certain front-back length of a product in order to reduce material cost.

It is desirable that an application amount is 0.08 to 0.10 g/10 cm × 10 cm.

The moisturizing agent M mainly containing glycerin can be applied to the outer surface of the intermediate sheet 40.

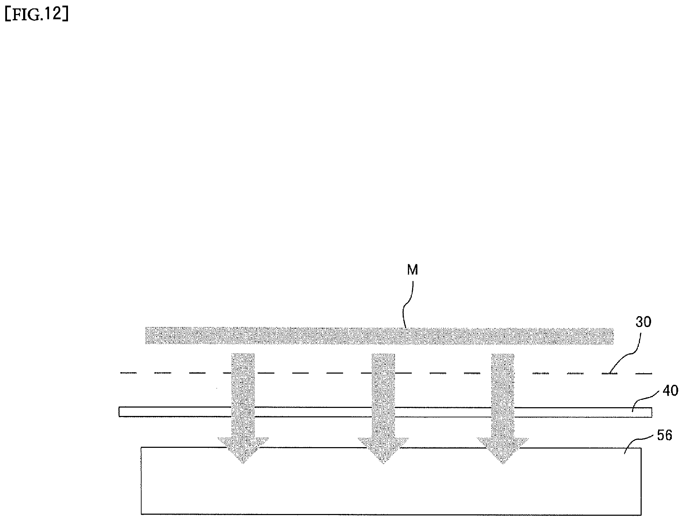

The moisturizing agent M is applied to the top sheet 30 by coating or the like in a diaper manufacturing process, and then a large number of diaper products are compactly packaged. Due to this consolidation of a diaper, as schematically illustrated in Fig. 12, a part of the moisturizing agent M of the top sheet 30 is transferred to the intermediate sheet 40 through the openings 14.

In the first place, the intermediate sheet has a higher body fluid permeation speed than the top sheet 30. In addition, the moisturizing agent M mainly containing glycerin has higher hydrophilicity than a hydrophilic diamide derivative. As a result, the moisturizing agent M that mainly contains glycerin and has been transferred to the intermediate sheet 40 side acts so as to draw in a body fluid from the top sheet 30 side or a loose stool component passing through the openings 14.

Therefore, the body fluid from the top sheet 30 side or the loose stool component passing through the openings 14 is quickly guided to the absorbent element 50 side, and remaining of the body fluid such as a loose stool component on a surface of the top sheet 30 is suppressed. As a result, stimulation received by the skin is reduced, and diaper rash can be suppressed.

Furthermore, the glycerin component of the moisturizing agent M moisturizes the skin and exhibits a skin care effect.

Furthermore, when the moisturizing agent M mainly containing glycerin is applied to an outer surface of the top sheet 30 (at least the outer surface portion contains the moisturizing agent M), the moisturizing agent M not only protects the skin of a wearer but also exhibits a function of reducing a frictional force with the skin of the wearer.

The moisturizing agent mainly containing glycerin according to the present invention contains 70% by mass or more of glycerin as a component composition, and contains one or more additives selected from the group consisting of an emulsifier, a phosphate, a paraffin, and a surfactant as an additive as necessary. As the surfactant, an ether type nonionic surfactant and a nonionic surfactant containing an EO/PO type are preferable.

The components of the embodiment will be described below.

### <Cover nonwoven fabric>

In many disposable absorbent articles such as a disposable diaper and a sanitary napkin, a configuration is known in which a liquid impervious sheet having air permeability is disposed on a back surface side of an absorber in order to secure air permeability while preventing back slipping of an absorbent liquid, and a back surface of the liquid impervious sheet is covered with a cover nonwoven fabric in order to impart a cloth-like appearance and texture to the liquid impervious sheet.

The cover nonwoven fabric 20 of the embodiment is also disposed in order to impart a cloth-like appearance and texture to the liquid impervious sheet. The cover nonwoven fabric 20 covers a back surface side of the liquid impervious sheet 11 and forms a product outer surface in at least a part of a portion covering the liquid impervious sheet 11.

In this case, when the cover nonwoven fabric is laminated on the air-permeable liquid impervious sheet, the air permeability is lowered by the presence of the cover nonwoven fabric. As a preferable method for solving this problem, a perforated nonwoven fabric having a large number of openings penetrating the nonwoven fabric from a front surface to a back surface is used as the cover nonwoven fabric.

Also in the illustrated embodiment, the cover nonwoven fabric 20 is a perforated nonwoven fabric in which the large number of openings 14 penetrating the nonwoven fabric from a front surface to a back surface are formed at intervals. The type of a fiber of the cover nonwoven fabric 20 and a processing method in connecting (interlacing) the fibers are not particularly limited, and can be appropriately selected. However, it is desirable to use an air through nonwoven fabric. In this case, the basis weight is preferably 20 to 30 g/m² and the thickness is preferably 0.3 to 1.0 mm.

In consideration of an air permeability improving effect, the cover nonwoven fabric 20 can have the openings 14 over the entire cover nonwoven fabric 20 in the front-back direction and the width direction in the tape-type disposable diaper.

### (Top sheet)

The top sheet 30 allows liquid to pass therethrough. Among the materials constituting the top sheet 30, the nonwoven fabric is not particularly limited concerning a raw material fiber thereof. Examples thereof include a synthetic fiber such as a polyolefin-based fiber including polyethylene and polypropylene, a polyester-based fiber, or a polyamide-based fiber, a regenerated fiber such as rayon or cupra, a natural fiber such as cotton, and a mixed fiber and a composite fiber in which two or more kinds of these fibers are used. Furthermore, the nonwoven fabric may be manufactured by any processing. Examples of a processing method include known methods such as a spunlace method, a spunbond method, a thermal bond method, a melt blown method, a needle punching method, an air through method, and a point bond method. For example, if softness and drapeability are demanded, a spunbond method and a spunlace method are preferable processing methods. If bulkiness and softness are demanded, an air through method, a point bond method, and a thermal bond method are preferable processing methods.

In particular, a nonwoven fabric manufactured by an air through method is desirable from a viewpoint of demanding bulkiness and softness.

As the nonwoven fabric fiber, for example, PE/PET of 1.5 to 3.5 dtex can be used.

It is desirable that the top sheet 30 has a basis weight of 10 to 30 g/m². If the basis weight is less than 10 g/m², returning of a body fluid may occur, and if the basis weight exceeds 30 g/m², it is difficult to obtain sufficient softness.

The top sheet 30 may be formed of a single sheet or a laminated sheet obtained by sticking two or more sheets to each other. Similarly, the top sheet 30 may be formed of a single sheet or two or more sheets in a plane direction.

Each side portion of the top sheet 30 may be folded back to a back surface side at a side edge of the absorbent element 50 or may protrude from the side edge of the absorbent element 50 to a lateral side without being folded back.

### <Planar arrangement of openings of top sheet>

In order to quickly transfer a body fluid to the absorbent element 50, the top sheet 30 is a perforated nonwoven fabric having openings 14. A planar arrangement of the openings 14 can be a regularly repeated arrangement such as an orthorhombic lattice as illustrated in Fig. 6(a), a hexagonal lattice as illustrated in Fig. 6(b) (these are also referred to as a staggered form), a square lattice as illustrated in Fig. 6(c), a rectangular lattice as illustrated in Fig. 6(d), or a parallel lattice as illustrated in Fig. 6(e) (a form in which two groups each including a large number of parallel and diagonal rows are disposed so as to cross each other as illustrated in the drawing).

### (Intermediate sheet)

Examples of the intermediate sheet 40 include a similar material to that of the top sheet 30, a spunlaced nonwoven fabric, a spunbonded nonwoven fabric, an SMS nonwoven fabric, a pulp nonwoven fabric, a mixed sheet of pulp and rayon, a point bonded nonwoven fabric, and crepe paper. In particular, an air through nonwoven fabric is preferable because of being bulky. As the air through nonwoven fabric, a composite fiber having a core-sheath structure is preferably used. In this case, a resin used for the core may be polypropylene (PP) but is preferably polyester (PET) having high rigidity. The basis weight is preferably 20 to 80 g/m², and more preferably 25 to 60 g/m². A raw material fiber of the nonwoven fabric preferably has a fineness of 2.0 to 10 dtex. In order to make the nonwoven fabric bulky, as mixed fibers of all or a part of raw material fibers, eccentric fibers having no core in the center, hollow fibers, eccentric and hollow fibers are also preferably used.

The intermediate sheet 40 in the illustrated form is disposed at the center so as to be shorter than the width of the absorber 56, but may be disposed over the maximum width. The front-back direction length of the intermediate sheet 40 may be the same as the maximum length of the diaper, may be the same as the length of the absorbent element 50, or may be within a short length range centered on a liquid receiving region.

For the purpose of preventing positional deviation with respect to a back surface side member or the like, it is desirable that the intermediate sheet 40 is fixed to a member adjacent to the back surface side by a bonding means by material welding such as heat sealing or ultrasonic sealing, or with a hot melt adhesive. In the illustrated embodiment, the intermediate sheet 40 is fixed to a surface of a portion located on a front surface side of the absorber 56 in the wrapping sheet 58 with a hot melt adhesive applied to a back surface thereof.

### (Liquid impervious sheet)

A material of the liquid impervious sheet 11 is not particularly limited, but examples thereof include a plastic film formed of a polyolefin-based resin such as polyethylene or polypropylene, a laminated nonwoven fabric having a plastic film disposed on a surface of a nonwoven fabric, and a laminated sheet obtained by superposing and bonding a nonwoven fabric or the like to a plastic film. For the liquid impervious sheet 11, it is preferable to use a liquid impervious and moisture pervious material favorably used from a viewpoint of preventing stuffiness. As a moisture pervious plastic film, a microporous plastic film obtained by kneading an inorganic filler in a polyolefin-based resin such as polyethylene or polypropylene, molding the kneaded material into a sheet, and then stretching the sheet in a monoaxial or biaxial direction is widely used. In addition, a nonwoven fabric using a micro denier fiber, and a sheet that has become liquid impervious without using a plastic film by a method for reinforcing leakproofness by reducing a space between fibers by applying heat and pressure, or a method for applying a highly water absorbing resin, a hydrophobic resin, or a water repellent agent, can be used as the liquid impervious sheet 11. However, it is desirable to use a plastic film in order to obtain a sufficient bonding strength at the time of bonding to the cover nonwoven fabric 20 described later through a hot melt adhesive.

The liquid impervious sheet 11 may have a width housed in a back surface side of the absorbent element 50 as illustrated in the drawing, or may go around both sides of the absorbent element 50 and extend to both side portions of a side surface of the top sheet 30 of the absorbent element 50 in order to enhance leakproofness. The extending portion appropriately has a width of about 5 to 20 mm on each of the left and the right.

On an inner side of the liquid impervious sheet 11, in particular, on a side surface of the absorber 56, an excretion indicator that changes a color due to absorption of a liquid can be disposed.

### (Side gather)

The side gather 60 extends over the entire front-back direction LD, is disposed in order to prevent side leakage by being in contact with a periphery of a wearer's leg, and includes what is generally called a three-dimensional gather 69 or a plane gather 61.

As the gather nonwoven fabric 62, a product obtained by subjecting a soft nonwoven fabric having excellent uniformity and concealability, such as a spunbonded nonwoven fabric (SS, SSS, or the like), an SMS nonwoven fabric (SMS, SSMMS, or the like), or a melt blown nonwoven fabric, to a water repellent treatment with silicone or the like as necessary can be used suitably. The gather nonwoven fabric 62 preferably has a fiber basis weight of about 10 to 30 g/m². As the elongated elastically stretchable member 63, a rubber thread or the like can be used. When a spandex rubber thread is used, the spandex rubber thread preferably has a fineness of 470 to 1240 dtex, and more preferably has a fineness of 620 to 940 dtex. The rubber thread preferably has a stretch rate of 150 to 350%, and more preferably has a stretch rate of 200 to 300% at the time of fixing. Note that the term "stretch rate" means a value obtained when a natural length is assumed to be 100%. As illustrated in the drawing, a waterproof film can be interposed between the two portions obtained by folding the gather nonwoven fabric 62, and in this case, the gather nonwoven fabric 62 can be partially omitted in a portion where the waterproof film is present. However, in order to impart a cloth-like appearance and texture to a product, at least an outer surface from a base edge of the side gather 60 to a tip thereof needs to be formed of the gather nonwoven fabric 62 as in the illustrated embodiment.

The number of the elongated elastically stretchable members 63 disposed in the free portion of the side gather 60 is preferably two to six, and more preferably three to five.

### (Absorbent element)

The absorbent element 50 includes the absorber 56 and the wrapping sheet 58 wrapping the entire absorber 56.

### (Absorber)

The absorber 56 can be formed by an assembly of fibers. As this fiber assembly, in addition to those obtained by accumulating short fibers such as fluff pulps or synthetic fibers, a filament assembly obtained by opening a tow (fiber bundle) of synthetic fibers such as cellulose acetate as necessary can also be used. When fluff pulps or a short fibers are accumulated, a fiber basis weight may be, for example, about 100 to 300 g/m². In a case of a filament assembly, a fiber basis weight may be, for example, about 30 to 120 g/m². In a case of a synthetic fiber, a fineness is, for example, 1 to 16 dtex, preferably 1 to 10 dtex, and more preferably 1 to 5 dtex. In a case of a filament assembly, the filament may be formed of a non-crimped fiber but is preferably formed of a crimped fiber. The degree of crimp of the crimped fibers can be, for example, about 5 to 75, preferably 10 to 50, and more preferably 15 to 50 per inch. A uniformly crimped fiber is often used. In the absorber 56, super absorbent polymer particles are preferably dispersed and held.

The absorber 56 may have a rectangular shape, but may have an hourglass shape including a front end portion, a back end portion, and a narrowing portion located therebetween and having a narrower width than the front end portion and the back end portion.

The size of the absorber 56 can be appropriately determined as long as the absorber 56 extends to the front, back, left, and right of the position of a urine outlet.

### (Super absorbent polymer particles)

The absorber 56 can contain super absorbent polymer particles partially or entirely. The super absorbent polymer particles include "powder" in addition to "particles". As the super absorbent polymer particles, those used for this type of disposable diaper can be used as they are. For example, when sieving using a standard sieve of 500 µm (JIS Z8801-1: 2006) (shake for five minutes) is performed, particles in which a ratio of particles remaining on the sieve is 30% by weight or less are desirable. When sieving using a standard sieve of 180 µm (JIS Z8801-1: 2006) (shake for five minutes) is performed, particles in which a ratio of particles remaining on the sieve is 60% by weight or more are desirable.

A material of the super absorbent polymer particles can be used without particular limitation, but those having a water absorption capacity of 40 g/g or more are suitable. Examples of the super absorbent polymer particles include a starch-based material, a cellulose-based material, and a synthetic polymer-based material. A starch-acrylic acid (salt) graft copolymer, a saponified product of a starch-acrylonitrile copolymer, a cross-linked product of sodium carboxymethyl cellulose, an acrylic acid (salt) polymer, or the like can be used. As the shapes of the super absorbent polymer particles, a usually used particulate material shape is suitable, but other shapes can also be used.

As the super absorbent polymer particles, those having a water absorption speed of 70 seconds or less, particularly 40 seconds or less are suitably used. When the absorption speed is too slow, so-called returning that liquid supplied into the absorber 56 returns out of the absorber 56 tends to occur.

As the super absorbent polymer particles, those having a gel strength of 1000 Pa or more are suitably used. This makes it possible to effectively suppress a sticky feeling after liquid absorption even in a case of using the bulky absorber 56.

The basis weight of the super absorbent polymer particles can be appropriately determined depending on the absorption amount required for an application of the absorber 56. Therefore, the basis weight can be 50 to 350 g/m² although this cannot be applied generally. The basis weight of a polymer of less than 50 g/m² makes it difficult to secure the absorption amount. The basis weight of more than 350 g/m² saturates an effect.

The spray density or the spray amount of the super absorbent polymer particles in a planar direction of the absorber 56 can be adjusted if necessary. For example, the spray amount at a liquid excretion site can be larger than that at another site. When a gender difference is considered, the spray density (amount) at a front side can be increased for men, and the spray density (amount) at a central portion can be increased for women. It is also possible to locally dispose a portion where no polymer is present (for example, in a spot shape) in a planar direction of the absorber 56.

### (Wrapping sheet)

The absorber 56 is covered with the wrapping sheet 58 in order to prevent escape of the super absorbent polymer particles or to improve shape maintenance of the absorber 56.

When the wrapping sheet 58 is used, as a material thereof, tissue paper, particularly, crepe paper, a nonwoven fabric, a polylaminated nonwoven fabric, a sheet with small holes, and the like can be used.

Conventionally, crepe paper has been often used. In the present invention, an SMS nonwoven fabric (spunbond-meltblown-spunbond laminated nonwoven fabric) or an SMMS nonwoven fabric (spunbond/meltblown/meltblown/spunbond laminated nonwoven fabric) is used.

When crepe paper is used, pulp fibers thereof are arranged in the front-back direction (MD (Machine Direction)) and are arranged densely. Therefore, rigidity in the front-back direction is particularly high (poor in softness).

On the other hand, when an SMS nonwoven fabric or an SMMS nonwoven fabric is used, rigidity in the front-back direction is particularly low (excellent in softness), and as illustrated with a cantilever test result described later, bending rigidity in the front-back direction (MD) and at an angle of 45 degrees indicates a lower value than that in a case of using crepe paper.

As a result, when the disposable diaper is worn, the wrapping sheet is favorably deformed (favorably bent) according to a change in the posture of a wearer, contact with the skin of the wearer is secured, and a leakage preventing effect is enhanced.

As materials of the SMS nonwoven fabric and the SMMS nonwoven fabric, polypropylene, a polyethylene/polypropylene composite material, or the like can be used. In particular, a nonwoven fabric that has been subjected to a hydrophilization treatment for enhancing body fluid absorbing characteristics is desirable.

A nonwoven fabric having a basis weight of 5 to 40 g/m², particularly of 10 to 30 g/m² is desirable.

A wrapping mode of the wrapping sheet 58 can be determined appropriately. However, a form is preferable in which the wrapping sheet 58 is wound around the absorber 56 cylindrically so as to surround front and back surfaces and both side surfaces of the absorber 56, front and back end portions of the wrapping sheet 58 are caused to protrude from the front and back of the absorber 56, and a wound and overlapping portion and an overlapping portion of the front and back protruding portions are bonded by a bonding means such as a hot melt adhesive or material welding from viewpoints of ease of manufacture, prevention of leakage of the super absorbent polymer particles from front and back edges, and the like.

If necessary, only the front and back surfaces may be covered with two nonwoven fabrics without covering both side surfaces of the absorber 56.

### <Explanation of terms in specification>

The following terms in the specification have the following meanings unless otherwise specified in the specification.

- "Front-back (longitudinal) direction" means a direction connecting a ventral side (front side) and a dorsal side (back side), and "width direction" means a direction orthogonal to the front-back direction (left-right direction).
- "Front surface side" means a side closer to a wearer's skin when a tape-type disposable diaper is worn. "Back surface side" means a side far from a wearer's skin when a tape-type disposable diaper is worn.
- "Front surface" means a surface of a member closer to a wearer's skin when a tape-type disposable diaper is worn. "Back surface" means a surface far from a wearer's skin when a tape-type disposable diaper is worn.
- "Area ratio" means the ratio of a target portion with respect to a unit area, and represents a ratio expressed by percentage, obtained by dividing the total area of a target portion (for example, holes) in a target region (for example, cover nonwoven fabric) by the area of the target region. In a form in which a large number of target portions are disposed at intervals, it is desirable to set the target region to a size that includes 10 or more target portions and to determine the area ratio. For example, the area ratio of a hole can be measured according to the following procedure using, for example, a trade name VHX-1000 manufactured by KEYENCE Corporation under measurement conditions of 20 times.
   (1) A sample is set to a lens having a magnification of 20, and the focus is adjusted. The position of a nonwoven fabric is adjusted such that 4 × 6 holes are included.
   (2) The brightness of the region of the holes is specified, and the area of the hole is measured.
   (3) Color extraction of "Area measurement" in "Measurement/Comment" is clicked. The portion of the holes is clicked.
   (4) "Collective measurement" is clicked, "Display measurement result window" is checked, and data is stored as CSV data.
- "Stretch rate" means a value obtained when a natural length is 100%.
- "Gel strength" is measured as follows. To 49.0 g of artificial urine (mixture of 2% by weight of urea, 0.8% by weight of sodium chloride, 0.03% by weight of calcium chloride dihydrate, 0.08% by weight of magnesium sulfate heptahydrate, and 97.09% by weight of deionized water), 1.0 g of a super absorbent polymer is added, and the resulting mixture is stirred with a stirrer. The gel thus generated is left in a thermohygrostat at 40°C x 60%RH for three hours. Thereafter, the temperature is returned to room temperature, and a gel strength is measured with a curdmeter (Curdmeter-MAX ME-500 manufactured by I. Techno Engineering Co., Ltd.).
- "Basis weight" is measured as follows. A sample or a test piece is predried and then left in a test chamber or an apparatus in a standard state (test location is at a temperature of 20 ± 5°C and a relative humidity of 65% or less) so as to have a constant weight. Predrying refers to causing a sample or a test piece to have a constant weight in an environment that a relative humidity is 10 to 25% and a temperature does not exceed 50°C. Note that fibers having an official moisture regain of 0.0% do not have to be predried. A sample of 200 mm × 250 mm (± 2 mm) in size is cut out from a test piece having a constant weight using a cutting template (200 mm × 250 mm, ± 2 mm). The weight of the sample is measured. The weight is multiplied by 20 to calculate the weight per square meter to be used as a basis weight.
- "Thickness" is automatically measured under conditions that a load is 10 gf/cm² and a pressing area is 2 cm² using an automatic thickness meter (KES-G5 handy compression measuring program).
- Water absorption capacity is measured in accordance with JIS K7223-1996 "Test method for water absorption capacity of highly water absorbing resin".
- Absorption speed is "time to end point" when JIS K7224-1996 "Test method for absorption speed of highly water absorbing resin" is performed using 2 g of super absorbent polymer and 50 g of physiological saline.
- "Unfolded state" means a flatly unfolded state without contraction or slackness.
- The size of each portion means a size not in a natural length state but in an unfolded state unless otherwise specified.
- When environmental conditions in a test and a measurement are not described, the test and the measurement are performed in a test room or an apparatus in a standard state (test location is at a temperature of 20 ± 5°C and a relative humidity of 65% or less).

### Industrial Applicability

The present invention can be used not only for a tape-type disposable diaper but also for a general pad-type disposable diaper and a general underpants-type disposable diaper, and can also be applied to a disposable absorbent article such as a sanitary napkin.

### Reference Signs List

- 11: Liquid impervious sheet
- 20: Cover nonwoven fabric
- 20H: Hot melt adhesive
- 14: Opening
- 15: Bonding point
- 30: Top sheet
- 40: Intermediate sheet
- 50: Absorbent element
- 56: Absorber
- 58: Wrapping sheet
- 60: Side gather
- 62: Gather nonwoven fabric
- LD: Front-back direction
- WD: Width direction

## Claims

1. A disposable absorbent article comprising: a top sheet constituting a usage-side surface; a liquid impervious sheet disposed on a back surface side; and an absorbent element interposed between the top sheet and the liquid impervious sheet, wherein
the top sheet is a perforated nonwoven fabric in which a large number of openings penetrating the nonwoven fabric from a front surface to a back surface are formed at intervals in a front-back direction and a width direction of a product,
a liquid pervious intermediate sheet is disposed on a back surface side of the top sheet,
the top sheet and the intermediate sheet are bonded to each other at a large number of bonding points by thermal bonding or ultrasonic bonding,
the openings each have an area of 1 to 30 mm²,
the bonding points each have an area of 0.12 to 20 mm², the area being smaller than the area of the opening,
a separation distance between the openings in a width direction is different from a separation distance between the bonding points in the width direction by 0.2 mm or more, and
a separation distance between the openings in a front-back direction is different from a separation distance between the bonding points in the front-back direction by 0.2 mm or more.

2. The disposable absorbent article according to claim 1, wherein
the separation distance between the openings in the width direction is 3.0 to 9.0 mm, and the separation distance between the openings in the front-back direction is 2.5 to 9.0 mm, and
the separation distance between the bonding points in the width direction is 2.0 to 7.0 mm, and the separation distance between the bonding points in the front-back direction is 2.5 to 8.0 mm.

3. The disposable absorbent article according to claim 1, wherein repetitive rows of the bonding points are inclined in each of the width direction and the front-back direction.

4. The disposable absorbent article according to claim 1, wherein a moisturizing agent mainly containing glycerin is applied to the top sheet.

5. The disposable absorbent article according to claim 4, wherein the moisturizing agent is applied in the front-back direction at intervals in the width direction.

6. The disposable absorbent article according to claim 1, wherein the intermediate sheet is a second sheet that is disposed between the top sheet and the absorbent element and has a higher body fluid permeation speed than the top sheet.
